# EUROPEAN PATENT APPLICATION

(11) **EP 4 183 437 A1**
(43) Date of publication of application: **24.05.2023**
(21) Application number: 21857687.4
(22) Date of filing: 17.08.2021
(51) Int. Cl.: A61M 16/04, A61B 1/267, A61B 1/04

(54) **VISUAL LARYNGOSCOPE/VISUAL GUIDE CORE-COMBINED DUAL-VISUALIZATION AND DUAL-POSITIONING TRACHEAL INTUBATION SET**

(30) Priority: 20.08.2020 CN 202021744864 U; 20.08.2020 CN 202010840687
(71) Applicant: Huang, Jiaqing, Xiamen, Fujian 361000 (CN)
(72) Inventor: WANG, Jinling, Xiamen, Fujian 361000 (CN); WANG, Ying, Xiamen, Fujian 361000 (CN); HUANG, Jiaqing, Xiamen, Fujian 361000 (CN)
(74) Representative: Cleanthous, Marinos
(86) International application number: PCT/CN2021/113131
(87) International publication number: WO 2022/037595

(57) **Abstract**

A dual-visible and dual-positioning endotracheal intubation set with a visual laryngoscope (1) and a visual guide core (2). When performing endotracheal intubation, an endotracheal tube is sleeved on a visual guide core (2), quick guidance can be achieved at the glottis position by means of a visual laryngoscope (1), and then a camera device (26) of the visual guide core (2) is used for precise secondary glottis positioning, and the visual guide core (2) can be used alone for endotracheal intubation for patients with difficulty in opening the mouth. The use of dual-visible and dual-positioning technology can overcome the shortcomings of the existing endotracheal intubation technology and achieve full visualization during the endotracheal intubation process, easy operation, fast intubation speed, and high success rate of one-time intubation.

## Description

### TECHNICAL FIELD

The present disclosure relates to the technical field of medical devices, and in particular to a dual-visible and dual-positioning endotracheal intubation set with a visual laryngoscope and a visual guide core, which is particularly suitable for fast intubation of patients with difficult airways.

### BACKGROUND

In clinical practice, for critically ill patients, especially those termination of respiration and those under general anesthesia, endotracheal intubation is required to help patients breathe. Endotracheal intubation refers to the technique of placing a special endotracheal tube into trachea via glottis, which can provide the optimum conditions for airway patency, ventilation and oxygen supply, respiratory tract suction and prevention of aspiration. For some critically ill patients and patients under general anesthesia, endotracheal intubation is a necessary means to maintain respiratory function, and medical staff must deliver the endotracheal tube into the trachea of the patient within a short time to provide respiratory support, so how to perform endotracheal intubation quickly and effectively has become a key issue for the treatment and rescue of patients. In clinical practice, some patients with difficulty in endotracheal intubation are often encountered, the use of common endotracheal intubation methods leads to prolonged endotracheal intubation or even failure of endotracheal intubation, which makes patients lose the best time for rescue, causing serious injuries to patients or even leading to their death and bringing medical risks to hospitals.

At present, the tools commonly used for clinical endotracheal intubation are: direct-vision laryngoscopes, visual laryngoscopes, visual guide cores, fiberoptic bronchoscopes, light wands, etc. However, these tools have certain limitations and shortcomings in clinical use. Despite the availability of adequate technology, due to anatomical variations and individual differences, endotracheal intubation for some difficult airways is hard to complete within a short time, or even fails, due to various reasons such as the inability of the physician to observe the situation surrounding the glottis, especially for physicians with insufficient technical experience in endotracheal intubation.

First, direct-vision laryngoscope: the direct-vision laryngoscope, also known as a direct laryngoscope, a fiberoptic laryngoscope or an ordinary laryngoscope, is low in cost and durable. Although it is widely used in clinic, there are also certain limitations and shortcomings in clinical use. 1. There is a certain requirement for the body position of patient, the patient is required to adopt a supine position with the head tilted back in a sniffing position during intubation, and at this time, the mouth axis, pharynx axis and larynx axis of the patient are in one straight line. For a small number of patients with difficult airways caused by special body types such as short thyromental distance, micrognathia, prominent incisors, tongue body hypertrophy, high larynx, obesity, stubby neck, limited mandibular mobility, limited cervical spine motion or the patients who cannot tilt the head back due to cervical spine injury, when three axes in one straight line cannot be achieved, only blind intubation can be performed as the glottises are often not visible during endotracheal intubation. Blind intubation is somewhat tentative, and repeated stimulation on the pharynx may cause local mucosal bleeding, edema or even laryngeal spasm and glottis closure, which may cause great damage to the airway and further increase the difficulty of endotracheal intubation. Blind intubation is prone to mistaken insertion into the esophagus, resulting in aspiration complications, low success rate of intubation or even failure of intubation. 2. To make three axes in one straight line to expose the glottis, the direct-vision laryngoscope often needs to expose the oropharynx excessively, which requires an increased lifting force of a laryngoscope handle and is prone to causing injuries to the lips, teeth, tongue and pharynx. 3. In the direct-vision laryngoscope, in order to expose the glottis, the laryngoscope needs to be lifted forcibly, which is prone to causing cardiovascular reaction during intubation. 4. The blade of direct-vision laryngoscope has a large thickness, and can be placed in the mouth only when the mouth opening is large (generally more than 3 cm), which is not suitable for patients with limited mouth opening (the mouth opening is less than 3 cm).

Second, visual laryngoscope: there are two types of visual laryngoscopes in clinical use. One of the visual laryngoscopes has the blade curvature similar to that of the direct-vision laryngoscope, except that the addition of visual devices, its intubation method is similar to that of the direct-vision laryngoscope to require the three axes to be in one straight line or close to be in one straight line. However, due to the existence of the visual device which is conducive to the physicians to observe the glottis, the advantages and disadvantages of this type of visual laryngoscope are the same as those of the direct-vision laryngoscope. The other visual laryngoscope has a large blade curvature, which is in line with the physiological curve of the oropharynx, making the visual angle turned forward by about 60 degrees (the design angles of different manufacturers are different) and the point of sight moved forward, so that endotracheal intubation can be performed without excessive back tilting of the head and neck, and the glottis can be exposed without making the mouth axis, pharynx axis and larynx axis in one straight line, thus the exposure of the glottis is simple. Many studies show that the visual laryngoscope can improve the glottis exposure of difficult airway by one to two grades or even three grades compared with the direct-vision laryngoscope, which solves the endotracheal intubation problem of most difficult airway under general anesthesia, and most clinically used visual laryngoscopes are of this type. The visual laryngoscopes described below in the present disclosure are of this type. The visual laryngoscope makes the glottis exposure easier, but also has certain limitations and shortcomings in clinical use: 1. It is easy to expose the glottis with the visual laryngoscope, but it is more complicated and difficult to intubate into the glottis than with the direct-vision laryngoscope, the reason is that, in order to prevent the front part of the blade from blocking the field of view of the lens and the tube from blocking the field of view of the lens when the endotracheal tube enters the field of view of the lens, the blade curvature is large, and thus the corresponding bending angle of the front end of the endotracheal tube must be large. When the endotracheal tube is inserted into the glottis, the mouth axis, pharynx axis and larynx axis are not in one straight line, but bend at a large angle, then the tip of the endotracheal tube first leans forward against the front wall of the laryngeal chamber under the glottis, and then bends downward to enter the glottis and trachea, which makes large intubation resistance, and the resistance of endotracheal intubation may cause the tip of the endotracheal tube to slip out of the glottis, thus the endotracheal tube cannot be smoothly placed into the glottis even if the tip-adjustable guide core is used. When the direct-vision laryngoscope is used for intubation, the eyes look at the outside of the mouth, and the line of sight looks at that the tip of the tube is directly inserted into the glottis, but the visual laryngoscope makes the line of sight moved to a display screen, as the human eyes are not consistent with the field of vision, what the eyes see is not the what wanted due to the parallax error. Therefore, although the field of vision of glottis can be seen easier, it is more difficult to determine the relationship between the tip of the tube and the glottis, which is prone to causing difficult intubation even mistaken insertion into the esophagus. 2. The large curvature of the blade of the visual laryngoscope makes the glottis exposure easy but intubation difficult, so the guide core of the visual laryngoscope generally employs a relatively hard guide wire or special hard guide core, but the over-hard guide core is prone to causing accident injury to the pharynx, larynx and glottis. 3. The visual laryngoscope can only be used to observe the oral cavity and the glottis position, and when performing endotracheal intubation, the endotracheal tube often blocks the field of view of the lens of the visual laryngoscope when entering the glottis, so the visualization effect cannot be fully achieved, and the endotracheal tube may be mistakenly inserted into the esophagus. 4. The glottis cannot be observed clearly as secretions of the patients with many oral secretions are adhered to the lens, which leads to low success rate of intubation. 5. The visual laryngoscope, like the direct-vision laryngoscope, has a large blade thickness, and can be placed in the mouth only when the mouth opening is large (generally more than 3 cm), which is not suitable for patients with limited mouth opening (the mouth opening is less than 3 cm).

Third, visual guide core: there are many visual guide core names in clinical application, such as visual tube cores, video light wands, tube type visual laryngoscopes. In the present disclosure, the guide core which is inserted into the endotracheal tube for guiding endotracheal intubation and is provided with a camera device is collectively referred to as a visual guide core. The visual guide cores commonly used in clinical practice include shapable visual guide cores and hard non-shapable visual guide cores. The hard non-shapable visual guide core is generally shaped in advance according to the physiological curve of oropharynx, and cannot be individually reshaped during use, its advantages are that the guide core is hard, cannot be deformed during intubation, and is not easy to be damaged because there is no need to bend and reshape the guide core repeatedly; while its disadvantages are that the guide core cannot be reshaped individually, and the hard guide core is prone to damaging the oral mucosa, epiglottis, glottis area, vocal cords and other tissues. The advantages and disadvantages of the shapeable visual guide core are opposite to those of the hard non-shapable visual guide cores. When performing endotracheal intubation, the lower incisor of the patient is grasped and the mandible and the tongue are lifted with the left thumb to enlarge the space of the pharyngeal cavity, the visual guide core is held with the right hand and then is inserted into the oral cavity for endotracheal intubation. If necessary, the assistant can help lift the mandible of patient to help the operator find the glottis behind epiglottis to expose. The visual guide core has a low requirement on the mouth opening, and can solve the endotracheal intubation problems of most patients with difficult airways who have difficulty in opening the mouth and small mouth opening, but it also has certain limitations and shortcomings in clinical use: 1. The method of grasping the lower incisor and lifting the mandible and tongue of the patient with the left thumb so as to enlarge the space of the pharyngeal cavity can be performed only on the premise that the patient has no occlusal ability. However, during actual clinical practice, especially for the emergency intubation, the patients without general anesthesia and muscle relaxation due to their illness still have spontaneous breathing and occlusal ability, at this time, the operator cannot put the thumb in the mouth of the patient, and the visual guide core intubation method cannot be used. 2. Due to the fact that the method of grasping the lower incisor and lifting the mandible and tongue of the patient with the left thumb is poor in effect of exposing the epiglottis and glottis, and the difficult degree of exposing the epiglottis and glottis of the patients under general anesthesia or the critical patients without spontaneous breathing is further increased due to the aggravation of muscle relaxation, the field of vision of inserting the visual guide core is narrow, the path is obstructed, and there is even no sufficient space for the entrance of the guide sometimes. At this time, even if the assistant helps to hold the mandible of the patient to facilitate the operator to expose the epiglottis and glottis, it is often impossible to fully expose the epiglottis and glottis, which makes the intubation operation difficult. 3. Endotracheal intubation with the visual guide core often fails to expose glottis due to the blocking by epiglottis, and the guide core needs to bypass the epiglottis from the epiglottis side or cross the epiglottis downwards. The large intubation difficulty in intubation operation results in prolonged intubation time, the endotracheal intubation cannot be complete rapidly or even fails. 4. The shapable visual guide cores are often deformed easily due to soft guide cores, which leads to the difficulty of intubation. The hard non-shapable visual guide cores are often not suitable for some patients due to the non-shapable performance, and meanwhile, the over-hard guide cores are prone to damaging oral mucosa, epiglottis, glottis area, vocal cords and other tissues during operation, resulting in bleeding or edema, which aggravates the difficulty of endotracheal intubation.

Fourth, light wand: as the light wand determines the glottis position only by the intensity of light transmission, it is a completely blind insertion, which is likely to cause injury to the lips, teeth, tongue, pharynx, glottis and trachea, and is more harmful to the human body. Moreover, it is not accurate to determine that the light wand has entered the glottis only by the brightness at the throat, the light wand may be mistakenly inserted into the esophagus, and the success rate of intubation is low.

Fifth, fiberoptic bronchoscope: the fiberoptic bronchoscope can be used for the endotracheal intubation of various difficult airways, but still has some limitation and shortcomings in clinical practice: 1. As the tube is soft, the direction adjustment in the oral cavity is time-consuming and labor-consuming, the operation is difficult, the operator is required to have rich clinical experience, and long operation time is required, thus the endotracheal intubation often cannot be completed quickly in emergency rescue. 2. The fiberoptic bronchoscope is not suitable for emergency endotracheal intubation to rescue patients because of its soft tube, difficult operation, long operation time, requirement of the sober cooperation of patients. 3. The fiberoptic bronchoscope equipment is complex, expensive, bulky, complicated in disinfection and high in application cost, and is not equipped in most departments in the hospital.

In conclusion, various endotracheal intubation methods above, for difficult airways of patients with special body types, are often difficult to complete endotracheal intubation in a short time or even result in failure of endotracheal intubation, and may cause damage to the patients and even make them lose the chance of rescue if a case of improper operation. Therefore, it is necessary to further improve the existing intubation tools to meet clinical needs, improve intubation speed and success rate, and reduce medical risks.

After successful endotracheal intubation, two problems are involved: 1. whether the endotracheal tube is located in the trachea or not; and 2. whether the insertion depth of the endotracheal tube is appropriate or not.

The following methods are commonly used in clinic to determine whether the endotracheal tube is in the trachea.
1. Gold standard: End-expiratory carbon dioxide waveforms and values: after endotracheal intubation, it is credible if the exhaled carbon dioxide can be continuously monitored in the first four breaths. However, there are some limitations and shortcomings in clinical practice: 1: Due to the fact that most monitors are not routinely installed with end-tidal carbon dioxide monitoring; after installation, it may take a long time to check the end-tidal carbon dioxide waveforms for four breaths, which may lead to serious and irreparable consequences if the endotracheal tube is mistakenly inserted into the esophagus. 2. Assuming that the ventilator assists ventilation according to the frequency of 12 times per minute, it may take 20 seconds to check the end-expiratory carbon dioxide waveforms of four breaths. If the endotracheal tube is mistakenly inserted into the esophagus, it takes longer to extubate and provide a ventilator mask to assist ventilation, and for critically ill and severely hypoxic patients, the lack of oxygen for such a long period of time may lead to serious and irreversible consequences. 3. For some patients with cardiac arrest, due to the lack of gas exchange, the carbon dioxide waveform cannot be displayed or the carbon dioxide pressure is particularly low even if the tube is in the trachea. 4. When the endotracheal tube is folded or obstructed, the balloon of the endotracheal tube is not inflated or ruptured, the carbon dioxide sampling tube is blocked or leaked or folded or disconnected, and severe bronchospasm occurs, the carbon dioxide waveform may not be displayed or the carbon dioxide pressure may be particularly low even if the endotracheal tube is in the trachea. 5. If there is mouth-to-mouth artificial respiration or balloon mask-assisted ventilation, exhaled breath may be blown into the stomach of patient, or the patient orally takes drugs or food containing carbonate for a short time, it may lead to the occurrence of carbon dioxide waveform or false positive in colorimetric detection device in the first few ventilations of the sampling of the endotracheal tube mistakenly inserted into the esophagus. Certainly, the waveform when the endotracheal tube is located in the esophagus can be found to be inconsistent with the waveform when the trachea endotracheal tube is located in the trachea by checking carefully, but it may take some time. 6. Special attention should be paid to the fact that the method cannot determine whether the insertion depth of the endotracheal tube is appropriate.
2. Regular mist of water inside the endotracheal tube with breathing: if the endotracheal tube is mistakenly inserted into the esophagus, there is mist but no regular movement with breathing. However, in actual clinical practice, it is not reliable to determine whether the endotracheal tube is in the trachea from the water vapor on the tube. Because if the gaseous distention is obvious, in fact, if the endotracheal tube is inserted into the esophagus, sometimes there is still a regular movement of the mist of water with breathing; and if the room temperature is high to lead to a small temperature difference between the exhaled gas in the endotracheal tube and the room temperature, or if the airway of the patient is dry, there is often no regular mist of water, or little mist of water, in the endotracheal tube with breathing.
3. Listening to breath sounds, and listening to upper abdomen contrast at the same time: it is correct only when the breath sounds in the lungs are strong and there is faint conduction sound in the upper abdomen; when listening to the breath sounds, the attention should be paid to alveolar sounds than only listening to the upper breast, the breath sound in the airway is prone to conduction, even if the endotracheal tube is inserted into the unilateral main bronchus, the breath sounds can often be heard in both lungs, but the alveolar sounds are definitely not audible. However, there are certain limitations and shortcomings in clinical use: 1. If the patient has spontaneous breathing, the alveolar sounds can be heard even if the endotracheal tube is mistakenly inserted into esophagus. 2. If the endotracheal tube is mistakenly inserted into the esophagus, if the stethoscope is placed near the endotracheal tube, sometimes breath sounds may still be heard and are mistaken for the fact that the endotracheal tube has been inserted into the trachea. 3. If the patient has evident emphysema or severe pulmonary lesions, the breath sounds are often not obvious, and the alveolar sounds are often inaudible, or the breath sounds are even incomprehensible. 4. Moist rales are often heard in the lungs of critically ill patients due to heart failure, lung infection, and other factors, and moist rales during auscultation of lungs may seriously interfere with the judgment of alveolar sounds.
4. Advanced technology, for example, the direct observation of endotracheal ring and carina by fiberoptic bronchoscope is a reliable index to determine whether the tube is located in trachea, and it is also very reliable to see the endotracheal tube between vocal cords by visual laryngoscope. However, the fiberoptic bronchoscope equipment is complex, expensive, bulky, complicated in disinfection, high in application cost, and is not equipped in most departments in the hospital. After endotracheal intubation and dental pad fixation, there is often no enough position in the oral cavity of the patient to place the visual laryngoscope. Even if the visual laryngoscope is placed into the oral cavity, it is also difficult to clearly see that the endotracheal tube is between the vocal cords due to the blocking of soft tissue around the vocal cords. Therefore, the visual laryngoscope cannot be applied to confirm whether the endotracheal tube is in the trachea in most cases.

The depth of endotracheal intubation for adults should be that the tip of the tube is about 3 cm to 5 cm above the carina. If the depth is excessive, the tip of the tube may be inwards shifted into the bronchus due to some reasons: for example, the pneumoperitoneum squeezes the diaphragm upward during laparoscopic gynecological surgery, and it has been found that the diaphragm may be shifted up by 3 cm to 5 cm at most. If the depth is shallow, the balloon may be stuck at the glottis to cause glottic injury, or the tube may slip out of the glottis due to the rotation or flexion of the head and neck. Therefore, the depth positioning needs to consider whether the patient may move the position of the head and neck, or whether the position of the diaphragm may be affected.

The following methods are commonly used in clinic to determine whether the insertion depth of the endotracheal tube is appropriate.
1. There are many methods for rough measurement according to the height, e.g., measuring the face of the patient with a tube to obtain an approximate length, or the distance from the tip of the nose to the earlobe plus half its length. In such a measurement, probably, for men, the height of 170 is taken as the median value, and the insertion depth is about 23 cm, and for women, the height of 160 is taken as the median value, and the insertion depth is about 21 cm.
2. Based on the anatomical data of the head and face, the insertion depth is calculated according to some formulas.
3. The distance from the palpebral fissure to the suprasternal notch of each individual is used as the actual intubation depth of each individual. The patient is required to lie horizontally without a pillow and close the eyes, then the length of the above distance is measured with a ruler.
4. It is suitable for children. The height of the children should also be proportional to the data of some anatomical marks on their body surfaces, and it is uncertain whether the above method is also suitable. The required insertion depth for the children may be estimated using the following formula: the depth of tube=(age+2)=12. However, even under the same age, the heights of the children vary greatly. Therefore, the intubation depth should be determined according to his own condition of each child. For young children, due to short trachea length, when the tube moves, it is easy to get out of the trachea or enter the bronchus. Someone proposed that, after intubation, the tube can be deliberately inserted into the unilateral bronchus over-deeply, then the tube can be withdrawn while listening to the breath sounds, and the tube needs to be withdrawn by 1 cm to 2 cm (according to the height) when the breath sounds of both lungs are symmetrical.
   The above four methods are empirical, and the depth of endotracheal insertion is often inappropriate due to individual differences. Therefore, the following methods are more commonly used in clinic.
5. Auscultation is performed on the axilla midline of both sides to determine whether the breathing movements of the two lungs are symmetrical. If the breath sounds of the left lung decrease after intubation, it is possible that the endotracheal tube is inserted into the right main bronchus, the endotracheal tube needs to be slowly withdrawn until the breath sounds of the two sides are symmetrical (that is, the left and right lungs are symmetrical).
6. After endotracheal intubation, chest fluoroscopy is used to check the lungs of the patient and confirm that the tip of an x-ray-opaque marking line on the endotracheal tube is in the middle of the trachea but not the left and right main bronchi. However, for the identification of the mistaken insertion into the esophagus, the x-ray fluoroscopy is unreliable.
7. Pulmonary CT has high accuracy. If the CT is used to check, it can be clearly identified that the endotracheal tube is in the trachea, and the insertion depth of the endotracheal tube can be determined at the same time. However, CT, only when performing examination, can be used to check the position and depth of endotracheal tube passingly, and CT is not specially used to determine the position and depth of the endotracheal tube in clinic.
8. The fiberoptic bronchoscope is the best to determine the insertion depth of the endotracheal intubation, but the fiberoptic bronchoscope equipment is complex, expensive, bulky, complicated in disinfection and high in application cost, and is not equipped in most departments in the hospital.

Various above methods for determining whether the endotracheal tube is located in the trachea as well as the insertion depth of the endotracheal tube is appropriate have certain shortcomings, and inappropriate insertion depth of the endotracheal tube, even the mistaken insertion of the endotracheal tube into the esophagus, may cause serious injury or even death to the patients. Therefore, it is necessary to further improve the existing intubation tools, so as to determine whether the endotracheal tube is located in the trachea as well as whether the insertion depth of the endotracheal tube is appropriate better, meet clinical needs, and reduce medical risks.

### SUMMARY

### Technical problem

At present, the tools commonly used for clinical endotracheal intubation are: direct-vision laryngoscopes, visual laryngoscopes, visual guide cores, fiberoptic bronchoscopes, light wands, etc. However, these tools have certain limitations and shortcomings in clinical use. Despite the availability of adequate technology, due to anatomical variations and individual differences, endotracheal intubation for some difficult airways is hard to completed within a short time, or even fails, due to various reasons such as the inability of the physician to observe the situation surrounding the glottis, especially for physicians with insufficient technical experience in endotracheal intubation, which makes patients lose the best time for rescue, causing serious injuries to patients or even leading to their death and bringing medical risks to hospitals. Therefore, it is necessary to further improve the existing intubation tools, so as to meet clinical needs, improve intubation speed and success rate of intubation, and reduce medical risks.

After successful endotracheal intubation, two problems are involved: 1. whether the endotracheal tube is located in the trachea or not; and 2. whether the insertion depth of the endotracheal tube is appropriate or not. At present, there are some shortcomings in various methods to determine whether the endotracheal tube is located in the trachea and whether the insertion depth of the endotracheal tube is appropriate: inappropriate insertion depth of the endotracheal tube, even the mistaken insertion of the endotracheal tube into the esophagus, may cause serious injury or even death to the patients. Therefore, it is necessary to further improve the existing intubation tools, so as to determine whether the endotracheal tube is located in the trachea as well as whether the insertion depth of the endotracheal tube is appropriate better, meet clinical needs, and reduce medical risks.

### Technical solution

To improve the existing intubation tools, improve the intubation speed and the success rate of intubation, and reduce the medical risk, a dual-visible and dual-positioning endotracheal intubation technology with a visual laryngoscope and a visual guide core is provided according to the present disclosure to overcome the shortcomings in the prior art, which is especially suitable for the rapid intubation of difficult airways. The trachea intubation is simpler and more convenient, the beginner can also master the trachea intubation rapidly, as well as rapidly determine whether the trachea intubation is located in the trachea and the insertion depth of the endotracheal tube is appropriate. The set is easy to master, can be conveniently promoted and popularized in medical staff, can overcome the shortcomings in the prior art and reduce the medical risks.

The set includes two parts: a visual laryngoscope 1 and a visual guide core 2.

The visual laryngoscope 1 includes a display screen 11, a handle 12, a laryngoscope blade 13, and a camera device 14.

The display screen 11 of the visual laryngoscope may receive images transmitted from the visual laryngoscope 1 and the visual guide core 2 simultaneously or alone. According to different models and different application scenarios, the display screen 11 may be fixed to the handle 12 of the visual laryngoscope or arranged outside the handle 12 alone to facilitate the observation. In a case that the display screen 11 is arranged outside the handle 12 alone, the display screen 11 may be connected to the handle 12 via a wireless device such as WiFi or Bluetooth for image transmission.

The handle 12 of the visual laryngoscope is provided with a switch, a data cable interface, a power module, and an integrated circuit. The data cable interface is connected to a power supply device or the display screen by a data cable. In a case that the display screen 11 is arranged outside the handle 12 alone, a wireless device such as WiFi or Bluetooth can be arranged in the handle 12 to send an image of the camera device to the display screen 11.

The tail part of the laryngoscope blade 13 of the visual laryngoscope is provided with the camera device 14, and illuminating lamps are arranged at the camera device side. A data cable of the camera device passes through the interior of the laryngoscope blade 13 to enter the handle 12, and is connected to the integrated circuit, so as to transmit images to the display screen 11 via the integrated circuit. The laryngoscope blade 13 is a blade bent in accordance with the physiological curvature of the oropharynx. In common visual laryngoscopes, in order to prevent the front part of the blade from blocking the field of view of the lens and prevent endotracheal tube from blocking the field of view of the lens when entering the field of view of the lens, the blade has a large curvature. By employing a dual-visible and dual-positioning method, an objective of the visual laryngoscope is to expose the glottis, while the observation of the tube entering the glottis during endotracheal intubation mainly depends on the visual guide core, without considering that the tube blocks the field of view of the lens when entering the field of view of the lens, so the curvature of the laryngoscope blade is smaller than that of the general visual laryngoscope. During endotracheal intubation, a supine position with the head tilted back in a sniffing position can be adopted, at this time, the mouth axis, pharynx axis and larynx axis of the patient are in one straight line, which solves the problem that the visual laryngoscope is easy to expose the glottis but more complicated and difficult in intubation into the glottis than direct-vision laryngoscope.

The visual guide cores 2 may be classified into shapable guide cores and hard non-shapable guide cores according to different models and different application scenarios. The guide core includes a handle 21, a hollow tube (including two parts: a handle end 22 of the hollow tube and a camera device end 23 of the hollow tube), a slide stopper 24, and a camera device 26. One end of the handle 21 of the visual guide core is connected to the handle end 22 of the hollow tube. A switch, a data cable interface, an integrated circuit and a power module are arranged in the handle 21, and the data cable interface is connected to a power supply device or a display screen by a data cable. According to different models and different application scenarios, the handle 21 and the display screen 11 of the visual laryngoscope are connected by the data cable for image transmission or are used for image transmission via a wireless device such as WiFi or Bluetooth. In a case that the handle 21 and the display screen 11 are in wireless connection, the wireless device such as WiFi or Bluetooth is arranged in the handle 21, so as to send an image of the camera device to the display screen 11.

The hollow tubes (each including two parts: the handle end 22 of the hollow tube and the camera device end 23 of the hollow tube) may be classified into shapable hollow tubes and a hard non-shapable hollow tubes according to different models and different application scenarios. The camera device end 23 of the hollow tube is provided with the camera device 26, illuminating lamps are arranged at the camera device side, and a data cable 25 of the camera device passes through the hollow tube and then is connected to the integrated circuit of the handle 21, and is configured to transmit an image to the display screen 11. The periphery of the hollow tube is sleeved with the slide stopper 24.

The slide sopper 24 is frustum-shaped, the big end of the slide stopper is close to the handle 21, and the distance between the outer end face of the hollow tube and the outer end face of the endotracheal tube is always kept at about 10 mm by the slide stopper, so that the camera device 26 is not exposed out of the endotracheal tube and the patient is prevented from being injured.

At present, the visual laryngoscope commonly used in clinic employs the blade with a large curvature, so that endotracheal intubation can be performed without excessive back tilting of the head and neck, and the glottis can be exposed easily. However, the visual laryngoscope is easy and clear to expose the glottis but more complicated and difficult in intubation into glottis than direct-vision laryngoscope. By employing a dual-visible and dual-positioning method in the present disclosure, an objective of the visual laryngoscope is to expose the glottis, while the observation of the tube entering the glottis during endotracheal intubation mainly depends on the visual guide core, without considering that the tube blocks the field of view of the lens when entering the field of view of the lens, so the curvature of the laryngoscope blade is smaller than that of the general visual laryngoscope. A sniffing position can be used during endotracheal intubation, the mouth axis, pharynx axis and larynx axis of the patient are in one straight line during intubation, the resistance of tube delivery is small in the endotracheal intubation, and even if the glottis cannot be seen clearly due to secretions adhered to the lens of the camera device, the endotracheal intubation can be performed under direct vision, thus the problem that the visual laryngoscope is easy to expose the glottis but more complicated and difficult in intubation into glottis than the direct-vision laryngoscope and the problem that the glottis cannot be seen clearly due to the secretions adhered to the lens are solved.

The laryngoscope blade of the visual laryngoscope is in line with the physiological curve of the oropharynx, making the visual angle turned forward by about 60 degrees and the point of sight moved forward, so that it is unnecessary to overemphasize the sniffing position for a small number of special patients with difficult airways, and the glottis can be exposed without making the mouth axis, pharynx axis and larynx axis in one straight line. Moreover, due to the visualization characteristic of the guide core, the glottis can be found when the guide core is placed under the epiglottis. The tube can be inserted when the glottis is opened, it is unnecessary to sufficiently expose the glottis using the laryngoscope. Therefore, the endotracheal intubation can be performed visually even if the visual laryngoscope cannot expose the glottis, which solves the problems that the direct-vision laryngoscope is difficult in glottis exposure and requires excessive exposure of the oropharynx. Of course, in this case, the endotracheal intubation may be relatively difficult, so as long as the situation of the patient allows, even if both the standard sniffing position as well as three axes of mouth, pharynx and larynx in one straight line cannot be achieved, the body position of the patient should be set well, so as to reduce the angle difference among the mouth axis, pharynx axis and larynx axis and reduce the delivery difficulty during endotracheal intubation. In accordance with the present disclosure, some shortcomings of the visual laryngoscope and the direct-vision laryngoscope can be avoided while making full use of the respective advantages of the visual laryngoscope and the direct-vision laryngoscope, the success rate of intubation may be remarkably improved, and the complications may be reduced.

By employing the dual-visible and dual- positioning method in the present disclosure, the glottis can be exposed with the visual laryngoscope, while the observation of the tube entering the glottis during endotracheal intubation mainly depends on the visual guide core, thus the problem that it is difficult for the visual laryngoscope to determine the relationship between the tip of the endotracheal tube and the glottis is solved. In accordance with the present disclosure, the glottis is exposed with the visual laryngoscope, the problem that a thumb of an operate needs to be put into the mouth of the patient when the endotracheal intubation is performed using the visual guide core and the problem that the epiglottis and the glottis are difficult to expose are solved. In accordance with the present disclosure, the glottis is exposed with the visual laryngoscope, the tongue body is pushed away by the visual laryngoscope when performing endotracheal intubation, and there is a large space for the visual guide core to enter the oral cavity, the injury is reduced, and the success rate of intubation is increased.

In accordance with the present disclosure, for some patients with difficulty in opening the mouth, the visual guide core can be used for endotracheal intubation alone, which solves the problem of endotracheal intubation for patients with difficulty in opening the mouth.

In accordance with the present disclosure, whether the endotracheal tube is located in the trachea and whether the insertion depth of the endotracheal tube is appropriate can be determined through the shapable visual guide core, and the problems that whether the endotracheal tube is located in the trachea and whether the insertion depth of the endotracheal tube is appropriate in clinic at present are solved.

The endotracheal intubation can be performed according to the following steps.
1. The endotracheal tube is sleeved on the visual guide core after the visual guide core is lubricated, the slide stopper on the visual guide core is adjusted to make a camera end of the visual guide core at the position in the endotracheal tube which is about 1 cm from the tip of the endotracheal tube, so that the camera device and other components cannot be exposed to damage the mucosal tissue of the patient, and can be prevented from making contact with dirt such as sputum and saliva to some extent, which may affect the image effect.
2. Power supplies of the visual laryngoscope and the visual guide core are turned on to determine that images transmitted by the visual laryngoscope and the visual guide core can be seen on the display screen simultaneously.
3. The patient is required to adopt a supine position with the head tilted back in the sniffing position. For a small number of patients with difficult airways caused by special body types such as short thyromental distance, micrognathia, prominent incisors, tongue body hypertrophy, high larynx, obesity, stubby neck, limited mandibular mobility, limited cervical spine motion or the patients who cannot tilt the head back due to cervical spine injury, it is unnecessary to overemphasize three axes in one straight line, the visual laryngoscope is held with the left hand, and three anatomic landmarks may be exposed in sequence by means of the visual laryngoscope: uvula, epiglottis, and glottis.
4. The endotracheal tube sleeved with the visual guide core is held with the right hand, and then is inserted into the oral cavity. The camera device of the visual guide core is configured to display the situation images in the oral cavity on the display screen in real time for an operator to perform secondary glottis positioning. The operator can push the endotracheal tube into the trachea when seeing the glottis, withdraw the visual guide core, adjust the depth of the endotracheal tube, and fix the endotracheal tube, so as to complete the intubation.
5. After the intubation and dental pad fixation are completed, it is possible to determine whether the endotracheal tube is located in the trachea and whether the insertion depth of the endotracheal tube is appropriate by the shapable visual guide core while a ventilator is configured to assist ventilation.
6. When the ventilator is configured to assist ventilation, if the patient has restlessness, shortness of breath, decreased blood oxygen saturation, etc., it is also possible to confirm whether the endotracheal tube is located in the trachea, whether the insertion depth of the endotracheal tube is appropriate, whether there is endotracheal tube hernia, whether there is main bronchial sputum and other problems causing main bronchial blockage through the shapable visual guide core while the ventilator is configured to assist ventilation.
7. For some patients having difficulty in opening the mouth, if the visual laryngoscope cannot be placed in the mouth of the patient, the visual guide core can be used alone for endotracheal intubation without using the laryngoscope. The endotracheal tube is sleeved on the visual guide core after the visual guide core is lubricated, the slide stopper on the visual guide core is adjusted; the power supplies of the display screen and the visual guide core are turned on to determine that the images transmitted by the visual guide core can be seen on the display screen. After the body position of the patient is set well, the endotracheal tube sleeved with the visual guide core is inserted into the oral cavity, the situation images in the oral cavity can be displayed on the display screen in real time by the camera device for the operator to find the glottis. Due to the visualization characteristic of the guide core, the glottis can be found when the guide core is placed under the epiglottis, the operator can push the endotracheal tube into the trachea when seeing the glottis, withdraw the visual guide core, adjust the depth of the endotracheal tube, and fix the endotracheal tube, so as to complete the intubation. This method has a low requirement on the mouth opening, even if the mounting opening is about 0.5 cm, and the endotracheal intubation of difficult airway becomes simple and accurate.

By using the dual-visible and dual-positioning endotracheal intubation technology with the visual laryngoscope and the visual guide core, quick guidance of the camera device of the visual laryngoscope can be achieved at the glottis position by the positioning of the visual laryngoscope, and the accurate secondary glottis positioning can be achieved by the camera device of the visual guide core, thus achieving rapid endotracheal intubation for the patient and ensuring the success rate of the endotracheal intubation. Moreover, whether the endotracheal tube is located in the trachea as well as whether the insertion depth of the endotracheal tube is appropriate can be determined by means of the visual guide core. The visual guide core can be used alone for endotracheal intubation for patients with difficulty in opening the mouth. The use of the dual-visible and dual-positioning endotracheal intubation technology with the visual laryngoscope and the visual guide core can achieve full visualization during the endotracheal intubation process, the risk of mistakenly inserting the tube into esophagus is avoided, the physician can determine the depth and position of the endotracheal intubation conveniently, the operability and security of the endotracheal intubation is greatly improved, and the medical risk is reduced.

The beneficial effects of the present disclosure are as follows:
1. By using the dual-visible and dual-positioning endotracheal intubation technology with the visual laryngoscope and the visual guide core, quick guidance of the camera device of the visual laryngoscope can be achieved at the glottis position by the positioning of the visual laryngoscope, and the accurate secondary glottis positioning can be achieved by the camera device of the visual guide core, thus achieving full and real-time visual guidance in the endotracheal intubation operation process, easy operation, fast intubation speed, and high success rate of one-time intubation. Risk of mistakenly inserting the tube into the esophagus is avoided, the operability and security of endotracheal intubation is greatly improved, the time effectiveness of intubation is effectively improved, and the medical risk is reduced. Therefore, the method is suitable for beginners to learn, and is suitable for clinical popularization and application.
2. Whether the endotracheal tube is located in the trachea and whether the insertion depth of the endotracheal tube is appropriate can be determined by the shapable visual guide core, and the problems that whether the endotracheal tube is located in the trachea and whether the insertion depth of the endotracheal tube is appropriate in clinic are solved.
3. In general visual laryngoscopes, in order to prevent the front part of the blade from blocking the field of view of the lens and prevent the endotracheal tube from blocking the field of view of the lens when the tube enters the field of view of the lens, so the blade has a large curvature. By employing a dual-visible and dual-positioning method, an objective of the visual laryngoscope is to expose the glottis, while the observation of the tube entering the glottis during endotracheal intubation mainly depends on the visual guide core, without considering that the tube blocks the field of view of lens when entering the field of view of the lens, so the curvature of the laryngoscope blade is smaller than that of the general visual laryngoscope. During endotracheal intubation, a supine position with the head tilted back in a sniffing position can be adopted, at this time, the mouth axis, pharynx axis and larynx axis of the patient are in one straight line, which solves the problem that the visual laryngoscope is easy to expose the glottis but more complicated and difficult in intubation into the glottis than direct-vision laryngoscope.
4. Due to the adoption of the sniffing position during endotracheal intubation, if the glottis cannot be seen clearly due to secretions adhered to the lens of the camera device, the endotracheal intubation can be performed under direct vision, thus solving the problem that the glottis cannot be seen clearly due to the secretions adhered to the lens.
5. When the direct-vision laryngoscope with a relatively straight laryngoscope blade is used for intubation, the eyes look at the outside of mouth, and the line of sight looks at that the tip of the tube is directly inserted into the glottis, while the visual laryngoscope makes the line of sight moved to a display screen, as the human eyes are not consistent with the field of vision, what the eyes see is not the what wanted due to the parallax error. Therefore, although the field of vision of glottis can be seen easier, it is more difficult to determine the relationship between the tip of the tube and the glottis, which is prone to causing difficult intubation even mistaken insertion into the esophagus. By using the dual-visible and dual-positioning method, the objective of the present disclosure is to expose the glottis, while the observation of the tube entering the glottis during endotracheal intubation mainly depends on the visual guide core, thus the problem that the visual laryngoscope is difficult to determine the relationship between the tip of the endotracheal tube and the glottis is solved.
6. The laryngoscope blade of the visual laryngoscope has a curvature in line with the physiological curve of the oropharynx, making the visual angle turned forward by about 60 degrees and the point of sight moved forward to facilitate the exposure of the glottis. Therefore, when a small number of patients with difficult airways caused by special body types such as short thyromental distance, micrognathia, prominent incisors, tongue body hypertrophy, high larynx, obesity, stubby neck, limited mandibular mobility, limited cervical spine motion or the patients who cannot tilt the head back due to cervical spine injury are encountered, although the mouth axis, the pharynx axis and the larynx axis of the patient are not in one straight line, the glottis still can be exposed as the field of vision of the laryngoscope is turned forward about 60 degrees and the sight of line is moved forwards, and the problem that the direct-vision laryngoscope is difficult in glottis exposure and requires excessive pharynx exposure is solved.
7. Due to the visualization characteristic of the guide core, the glottis can be found when the guide core is placed under the epiglottis, the endotracheal tube can be inserted when the glottis is opened, and the endotracheal intubation can be performed visually without fully exposing the glottis with the laryngoscope, thus solving the endotracheal intubation problem that the glottis of a few patients with difficult airways cannot be exposed even with the visual laryngoscope.
8. The glottis is exposed with the visual laryngoscope, thus the problem that the thumb needs to be put into the mouth of the patient and the problem of difficult exposure of the epiglottis and glottis when the endotracheal intubation is performed using the visual guide core are solved.
9. The glottis is exposed with the visual laryngoscope, the tongue body can be pushed away by the visual laryngoscope when performing the endotracheal intubation, so that there is a large space for the visual guide core to enter, the injury is reduced, and the success rate of intubation is increased.
10. For some patients with difficulty in opening the mouth, the visual guide core can be used alone for endotracheal intubation, and thus the problem of endotracheal intubation for the patients with difficulty in opening the mouth is solved.

### BRIEF DESCRIPTION OF THE DRAWINGS

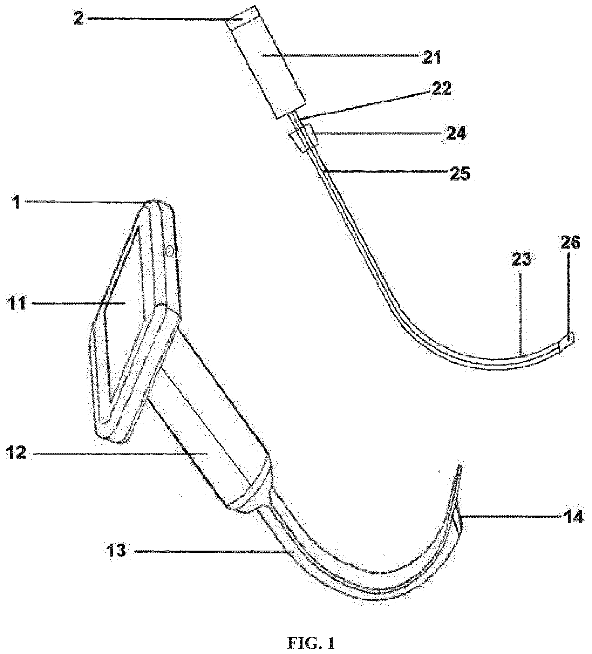

Fig. 1 is a structure diagram in accordance with the present disclosure.

In the drawings: 1-visual laryngoscope; 11-display screen; 12-handle of visual laryngoscope; 13-blade of visual laryngoscope; 14-camera device of visual laryngoscope; 2-visual guide core; 21-handle of visual guide core; 22-handle end of hollow tube; 23-camera device end of hollow tube; 24-slide stopper; 25-data cable of camera device of visual guide core; 26-camera device of visual guide core.

### DETAILED DESCRIPTION OF THE EMBODIMENTS

By using the dual-visible and dual-positioning endotracheal intubation technology with the visual laryngoscope and the visual guide core according to the present disclosure, different endotracheal intubation methods can be adopted according to different situations of patients, there is no optimal embodiment for all patients, but the first of the following specific embodiments is a better one for most patients without difficult airway.

Embodiments of the present disclosure are as follows:
By using the dual-visible and dual-positioning endotracheal intubation technology with the visual laryngoscope and the visual guide core according to the present disclosure, different endotracheal intubation methods can be adopted according to different situations of patients.

A first embodiment is as follows: for most of patients without difficult airway, the glottis can be clearly exposed with a direct-vision laryngoscope, a supine position with the head tilted back in a sniffing position is adopted during endotracheal intubation, and an mouth axis, a pharynx axis and a larynx axis of the patient are in one straight line during intubation: 1. A endotracheal tube is sleeved on a shapable visual guide core after the visual guide core is lubricated, a slide stopper on the visual guide core is adjusted and the visual guide core is shaped to keep a camera end of the shapable visual guide core at the position in the endotracheal tube which is about 1 cm from the tip of a endotracheal tube, so that the camera device and other components cannot be exposed to damage the mucosal tissue of the patient, and can be prevented from making contact with dirt such as sputum and saliva to some extent, which may affect the image effect. 2. Power supplies of a visual laryngoscope and the visual guide core are turned on to determine that images transmitted by the visual laryngoscope and the visual guide core can be seen simultaneously on the display screen. 3. The visual laryngoscope is used in the sniffing position, and the mouth axis, the pharynx axis and the larynx axis of the patient are overlapped by means of the change of the head and the body position, and at the moment, the visual laryngoscope is held with the left hand, and three anatomic landmarks may be exposed in sequence by means of the visual laryngoscope: uvula, epiglottis, and glottis. The endotracheal intubation may also be performed under direct vision even if the glottis cannot be seen clearly due to secretions adhered to the lens. 4. The endotracheal tube sleeved with the visual guide core is held with the right hand, and then is inserted into the oral cavity. A camera device of the visual guide core is configured to display the situation images in the oral cavity on the display screen in real time for an operator to accurately find the glottis for the second time; the operator can push the endotracheal tube into the trachea when seeing the open of the glottis, withdraw the visual guide core, adjust the depth of the endotracheal tube, and fix the endotracheal tube, so as to complete the intubation. 5. After the intubation is finished, a dental pad is fixed, and a ventilator is connected to assist ventilation, then whether the endotracheal tube is located in the trachea and whether the insertion depth of the endotracheal tube is appropriate can be determined by the shapable visual guide core while a ventilator is configured to assist ventilation. 6. When the ventilator is configured to assist ventilation, if the patient has restlessness, shortness of breath, decreased blood oxygen saturation, etc., it is also possible to confirm whether the endotracheal tube is located in the trachea, whether the insertion depth of the endotracheal tube is appropriate, whether there is endotracheal tube hernia, whether there is main bronchial sputum and other problems causing main bronchial blockage through the shapable visual guide core while the ventilator is configured to assist ventilation.

A second embodiment is as follows: For a small number of patients with difficult airways caused by special body types such as short thyromental distance, micrognathia, prominent incisors, tongue body hypertrophy, high larynx, obesity, stubby neck, limited mandibular mobility, limited cervical spine motion or the patients who cannot tilt the head back due to cervical spine injury, it is unnecessary to overemphasize three axes in one straight line as the direct-vision laryngoscope cannot expose the glottis clearly: 1. An endotracheal tube is sleeved on a shapable visual guide core after the visual guide core is lubricated, a slide stopper on the visual guide core is adjusted and the visual guide core is shaped to keep a camera end of the shapable visual guide core at the position in the endotracheal tube which is about 1 cm from the tip of the endotracheal tube, so that the camera device and other components cannot be exposed to damage the mucosal tissue of the patient, and can be prevented from making contact with dirt such as sputum and saliva to some extent, which may affect the image effect. 2. Power supplies of a visual laryngoscope and the visual guide core are turned on to determine that images transmitted by the visual laryngoscope and the visual guide core can be seen simultaneously on the display screen. 3. As long as the situation of the patient allows, the endotracheal intubation can be performed by using the sniffing position, even if both the standard sniffing position as well as the mouth axis, pharynx axis and larynx axis in one straight line cannot be achieved, the body position of the patient should be set well, so as to reduce the angle difference among the mouth axis, pharynx axis and larynx axis and reduce the delivery difficulty during endotracheal intubation. However, if the patient cannot use the sniffing position due to the reasons such as cervical spine injury, the patient can be kept at a safe body position. 4. The visual laryngoscope is held with the left hand, and three anatomic landmarks may be exposed in sequence by means of the visual laryngoscope: uvula, epiglottis, and glottis; the endotracheal tube sleeved with the visual guide core is held with the right hand, and then is inserted into the oral cavity. A camera device of the visual guide core is configured to display the situation images in the oral cavity on the display screen in real time for an operator to accurately find the glottis for the second time; the operator can push the endotracheal tube into the trachea when seeing the open of the glottis, withdraw the visual guide core, adjust the depth of the endotracheal tube, and fix the endotracheal tube, so as to complete the intubation. 5. After the intubation is finished, dental pad is fixed, and a ventilator is connected to assist ventilation, then whether the endotracheal tube is located in the trachea and whether the insertion depth of the endotracheal tube is appropriate can be determined by the shapable visual guide core while a ventilator is configured to assist ventilation. 6. When the ventilator is configured to assist ventilation, if the patient has restlessness, shortness of breath, decreased blood oxygen saturation, etc., it is also possible to confirm whether the endotracheal tube is located in the trachea, whether the insertion depth of the endotracheal tube is appropriate, whether there is endotracheal tube hernia, whether there is main bronchial sputum and other problems causing main bronchial blockage through the shapable visual guide core while the ventilator is configured to assist ventilation.

A third embodiment is as follows: for a few patients with difficult airways whose glottis cannot be exposed with the visual laryngoscope, as long as the situation of the patient allows, the endotracheal intubation can be performed by using the sniffing position, the body position of the patient should be set well, so as to reduce the angle difference among the mouth axis, pharynx axis and larynx axis and reduce the delivery difficulty during endotracheal intubation: 1. An endotracheal tube is sleeved on a shapable visual guide core after the visual guide core is lubricated, a slide stopper on the visual guide core is adjusted and the visual guide core is shaped to keep a camera end of the shapable visual guide core at the position in the endotracheal tube which is about 1 cm from the tip of the endotracheal tube, so that the camera device and other components cannot be exposed to damage the mucosal tissue of the patient, and can be prevented from making contact with dirt such as sputum and saliva to some extent, which may affect the image effect. 2. Power supplies of a visual laryngoscope and the visual guide core are turned on to determine that images transmitted by the visual laryngoscope and the visual guide core can be seen simultaneously on the display screen. 3. As long as the situation of the patient allows, the endotracheal intubation can be performed by using the sniffing position, even if both the standard sniffing position as well as the mouth axis, pharynx axis and larynx axis in one straight line cannot be achieved, the body position of the patient should be set well, so as to reduce the angle difference among the mouth axis, pharynx axis and larynx axis and reduce the delivery difficulty during endotracheal intubation. However, if the patient cannot use the sniffing position due to the reasons such as cervical spine injury, the patient can be kept at a safe body position. 4. The visual laryngoscope is held with the left hand to expose uvula and epiglottis in sequence, and for the patients whose glottis even the uvula and epiglottis cannot be exposed, the determination of intubation entering the glottis mainly depends on the visual guide core. The endotracheal tube sleeved with the visual guide core is held with the right hand, and then is inserted into the oral cavity. A camera device of the visual guide core is configured to display the situation images in the oral cavity on the display screen in real time for an operator to find the glottis. If the glottis cannot be exposed due to the blocking of the epiglottis, the visual guide core needs to bypass from the epiglottis side or cross the epiglottis downwards. Due to the visualization characteristic of the guide core, the glottis can be found by placing the guide core under the epiglottis, and it is unnecessary to fully expose the glottis with the laryngoscope. The operator can push the endotracheal tube into the trachea when seeing the open of the glottis, withdraw the visual guide core, adjust the depth of the endotracheal tube, and fix the endotracheal tube, so as to complete the intubation. If the glottis cannot be exposed as the shapable guide core is soft, the shapable guide core can be replaced with the hard non-shapable guide core to complete endotracheal intubation. 5. After the intubation is finished, a dental pad is fixed, and a ventilator is connected to assist ventilation, then whether the endotracheal tube is located in the trachea and whether the insertion depth of the endotracheal tube is appropriate can be determined by the shapable visual guide core while a ventilator is configured to assist ventilation. 6. When the ventilator is configured to assist ventilation, if the patient has restlessness, shortness of breath, decreased blood oxygen saturation, etc., it is also possible to confirm whether the endotracheal tube is located in the trachea, whether the insertion depth of the endotracheal tube is appropriate, whether there is endotracheal tube hernia, whether there is main bronchial sputum and other problems causing main bronchial blockage through the shapable visual guide core while the ventilator is configured to assist ventilation.

A fourth embodiment is as follows: For some patients with difficult airways who have difficulty in opening the mouth, a visual laryngoscope cannot be put in the mouth, a visual guide core can be used alone to perform endotracheal intubation without using the laryngoscope, a sniffing position can be adopted during endotracheal intubation, and it is unnecessary to overemphasize the sniffing position for a few special patients with difficult airways, but the angle difference among the mouth axis, the pharynx axis and the larynx axis should be reduced as much as possible, so as to reduce the delivery difficulty in endotracheal intubation: 1. An endotracheal tube is sleeved on a shapable visual guide core after the visual guide core is lubricated, a slide stopper on the visual guide core is adjusted and the visual guide core is shaped to keep a camera end of the shapable visual guide core at the position in the endotracheal tube which is about 1 cm from the tip of the endotracheal tube, so that the camera device and other components cannot be exposed to damage the mucosal tissue of the patient, and can be prevented from making contact with dirt such as sputum and saliva to some extent, which may affect the image effect. 2. Power supplies of a display screen and the visual guide core are turned on to determine that images transmitted by the visual guide core can be seen on the display screen. 3. As long as the situation of the patient allows, the endotracheal intubation can be performed by using the sniffing position, even if both the standard sniffing position as well as the mouth axis, pharynx axis and larynx axis in one straight line cannot be achieved, the body position of the patient should be set well, so as to reduce the angle difference among the mouth axis, pharynx axis and larynx axis and reduce the delivery difficulty during endotracheal intubation. However, if the patient cannot use the sniffing position due to the reasons such as cervical spine injury, the patient can be kept at a safe body position. 4. When performing endotracheal intubation, the lower incisor of the patient is grasped and the mandible and the tongue body are lifted with the left thumb to enlarge the space of the pharyngeal cavity, the endotracheal tube sleeved with the visual guide core is held with the hand and then is inserted into the oral cavity. If necessary, an assistant can help lift the mandible of patient to help the operator find the glottis behind epiglottis to expose. If the glottis cannot be exposed due to the blocking of the epiglottis, the visual guide core needs to bypass from the epiglottis side or cross the epiglottis downwards. Due to the visualization characteristic of the guide core, the glottis can be found by placing the guide core under the epiglottis, and it is unnecessary to fully expose the glottis with the laryngoscope. The operator can push the endotracheal tube into the trachea when seeing the open of the glottis, withdraw the visual guide core, adjust the depth of the endotracheal tube, and fix the endotracheal tube, so as to complete the intubation. If the glottis cannot be exposed as the shapable guide core is soft, the shapable guide core can be replaced with the hard non-shapable guide core to complete endotracheal intubation. 5. After the intubation is finished, dental pad is fixed, and a ventilator is connected to assist ventilation, then whether the endotracheal tube is located in the trachea and whether the insertion depth of the endotracheal tube is appropriate can be determined by the shapable visual guide core while a ventilator is configured to assist ventilation. 6. When the ventilator is configured to assist ventilation, if the patient has restlessness, shortness of breath, decreased blood oxygen saturation, etc., it is also possible to confirm whether the endotracheal tube is located in the trachea, whether the insertion depth of the endotracheal tube is appropriate, whether there is endotracheal tube hernia, whether there is main bronchial sputum and other problems causing main bronchial blockage through the shapable visual guide core while the ventilator is configured to assist ventilation. Such a method has a low requirement on mouth opening, and the endotracheal intubation for difficult airway becomes simple and accurate even if the mouth opening is only 0.5 cm.

The present disclosure is not limited to the above-mentioned specific embodiments, but extends to various modifications of the dual-visible and dual-positioning endotracheal intubation technology with a visual laryngoscope and a visual guide core in the appended claims. It should be understood by those skilled in the art that the technical solution of the present disclosure can be modified or equivalently replaced without departing from the spirit and scope of the technical solution of the present disclosure, and the modification and equivalent replacement should be included in the scope of the dual-visible and dual-positioning endotracheal intubation technology with the visual laryngoscope and the visual guide core of the present disclosure.

Industrial applicability is as follows:
At present, the clinical visual laryngoscope is widely used, and the visual guide core is also used in a small part. It is easy to transmit images of the visual laryngoscope and the visual guide core to the same display screen at the current industrial level, so the industrial practicability is achieved.

## Claims

1. An endotracheal intubation set, comprising a visual laryngoscope and a visual guide core, wherein the visual laryngoscope and the visual guide core are two separated components to facilitate the holding of the visual laryngoscope with one hand and the holding of an endotracheal tube sleeved with the visual guide core with the other hand when performing endotracheal intubation; a display screen, as a part of the visual laryngoscope, is fixed to a handle of the visual laryngoscope or is connected to the visual laryngoscope via a wireless device such as WiFi or Bluetooth for image transmission, and the visual guide core and the display screen are connected by a data cable or are configured for image transmission via the wireless device such as WiFi or Bluetooth.

2. The endotracheal intubation set according to claim 1, wherein the set adopts a dual-visible and dual-positioning endotracheal intubation technology with a visual laryngoscope and a visual guide core.

3. The endotracheal intubation set according to claim 1, wherein the visual laryngoscope of the set comprises a display screen, a handle, a laryngoscope blade, and a camera device; the visual laryngoscope is held with the left hand when performing endotracheal intubation, and three anatomic landmarks of uvula, epiglottis and glottis can be exposed in sequence by means of the visual laryngoscope, and the visual laryngoscope is used to expose, observe and position the glottis.

4. The endotracheal intubation set according to claim 1, wherein the visual guide core of the set comprises a handle, a hollow tube, a slide stopper, and a camera device; the visual guide cores are able to be classified into shapable visual guide cores and hard non-shapable visual guide cores according to different models and different application scenarios; when performing endotracheal intubation, the endotracheal tube is sleeved on the visual guide core after the visual guide core is lubricated, the slide stopper on the visual guide core is adjusted to enable a camera end of the visual guide core at the position in the endotracheal tube which is about 1 cm from the tip of the endotracheal tube; after the visual laryngoscope is held with the left hand to expose and position the glottis, the endotracheal tube sleeved with the visual guide core is held with the right hand, and the secondary glottis positioning is performed when seeing the glottis, then the endotracheal tube is pushed into the trachea; and after endotracheal intubation, the structure and tissue of the trachea are able to be observed by means of the visual guide core for trachea positioning; and the visual guide core is used to observe the full endotracheal intubation process, and observe and position the glottis and trachea.

5. The endotracheal intubation set according to claim 1, wherein the display screen, as a part of the visual laryngoscope, is fixed to the handle of the visual laryngoscope or arranged outside the handle of the visual laryngoscope alone to facilitate the observation; when the display screen is arranged outside the handle of the visual laryngoscope alone, the display screen is able to be connected to the handle of the visual laryngoscope via a wireless device such as WiFi or Bluetooth for image transmission; the handle of the visual guide core and the display screen are connected by a data cable for image transmission, or are configured for image transmission via a wireless device such as WiFi or Bluetooth for image transmission; images transmitted by the visual laryngoscope and the visual guide core are able to be displayed on the display screen simultaneously or alone, thus facilitating the image observation when the endotracheal intubation is performed using the dual-visible and dual-positioning technology.

6. The endotracheal intubation set according to claim 1, wherein the laryngoscope blade of the visual laryngoscope of the set is in a curvature in line with the physiological curve of the oropharynx, the curvature of the laryngoscope blade is smaller than that of the general visual laryngoscope to facilitate the endotracheal intubation when a sniffing position is adopted.

7. The endotracheal intubation set according to claim 1, wherein the shapable visual guide core is used to determine whether the endotracheal tube is located in the trachea as well as whether the insertion depth of the endotracheal tube is appropriate after the endotracheal intubation is completed.
